# EUROPÄISCHE PATENTSCHRIFT

(11) **EP 0 453 898 B1**
(45) Veröffentlichungstag und Bekanntmachung des Hinweises auf die Patenterteilung: **31.05.1995**
(21) Anmeldenummer: 91105829.5
(22) Anmeldetag: 12.04.1991
(51) Int. Cl.: A61K 39/395

(54) **Verwendung von anti-TNF-Antikörpern als Arzneimittel bei der Behandlung von Ischämien und deren Folgen**
Use of TNF-specific antibodies as a drug for treatment of ischemias and of their consequences
Utilisation d'anticorps contre le facteur de nécrose tumorale (TNF) comme médicament pour le traitement d'ischémies et de leurs conséquences

(30) Priorität: 25.04.1990 DE 4013114; 27.11.1990 DE 4037604
(43) Veröffentlichungstag der Anmeldung: 30.10.1991
(73) Patentinhaber: BAYER AG, 51368 Leverkusen (DE)
(72) Erfinder: Eckert, Peter, Prof. Dr., W-5300 Bonn 1 (DE); Schramm, Matthias, Dr., W-5090 Leverkusen 1 (DE)

(56) Entgegenhaltungen:
- EP-A- 351 789
- WO-A-89/08460
- TRANSPLANTATION, Band 49, Nr. 2, Februar 1990, Baltimore, MD (US); L. COLLETTI et al., Seiten 268-272/
- SCIENCE, Band 249, Nr. 4964, 06 Juli 1990, Washington, DC (US); A. LEFER et al., Seiten 61-64/
- JOURNAL OF CLINICAL INVESTIGATION, Band 85, Nr. 6, Juni 1990, New York, NY (US); L. COLLETTI et al., Seiten 1936-1943/

## Beschreibung

Die vorliegende Erfindung betrifft die Verwendung von monoklonalen anti-TNF-Antikörpern zur Behandlung von akutem Herzinfarkt und den damit verbundenen Begleiterscheinungen, wie Gewebsschädigungen, insbesondere ihre Verwendung in Arzneimitteln zur Arrhythmienreduktion, bei Herzinfarkten, Infarktgrößenreduktion und zur Erhöhung der Überlebensrate nach Myokard-Infarkt.

Es ist bekannt, daß der Begriff Tumor Necrosis Faktor (TNF) zwei zytotoxische Faktoren (TNF-α und TNF-β) umfaßt, die größtenteils von aktivierten Lymphozyten und Monozyten gebildet werden. Außerdem ist bekannt, daß bei bakteriellen Infektionen freigesetztes TNF einen Rückgang des Tumors bei Krebserkrankungen bewirkt.

Es wurde in Untersuchungen festgestellt, daß einige Erkrankungen, wie Parasiten-Infektionen, alkoholbedingte Lebererkrankungen, Hepatitis, Pl.-Falciparum-Malaria, rheumatoide Arthritis, Nierentransplantationen, Multiple Sklerose und Verletzungen mit einer starken Erhöhung des TNF-Spiegels verbunden sind, die oft lebensbedrohend werden kann [vgl. Z.A. Bharat, B. Aggarwal in "Drugs of the Future", 12 (1987), S. 891 ff.; American Journal of Medicine 87/2 (139-143), 1989; Transplantation 47 (4), 606-608, 1989].

In der Publikation EP 260 010 werden beispielsweise Anti-TNF-Antikörper publiziert, die bei Krankheiten wie sept. Schock, Transplantatabstoßung, Allergien, Autoimmunkrankheiten, Schocklunge, Blutgerinnungsstörungen oder entzündlichen Knochenerkrankungen, die mit einer Erhöhung von TNF im Blut verbunden sind, zur Inaktivierung eingesetzt werden können.

Ebenso werden in der EP 230 574 Antikörper gegen TNF beschrieben, die zur Behandlung von LAV/HTV III-Virus-Infektionen eingesetzt werden können.

Überraschenderweise wurde nun gefunden, daß Antikörper gegen TNF bei der Behandlung von akutem Herzinfarkt und dessen Folgen eingesetzt werden können. So kommt es bei Infarkten insbesondere nach Reperfusion oft zu massiven Gewebeschädigungen, die vielfach zum Tode führen. Durch Gabe von Antikörpern gegen TNF läßt sich der Gewebeschaden begrenzen und die Letalitätsquote senken.

Geeignet sind monoklonale Antikörper gegen TNF zur Behandlung von akutem Herzinfarkt und den damit verbundenen Arrhythmien und Gewebsschädigungen, insbesondere zur Verwendung in Arzneimitteln zur Arrhythmienreduktion, Infarktgrößenreduktion und zur Erhöhung der Überlebensrate nach Myokard-Infarkt.

Die monoklonalen Antikörper und die Verfahren zu ihrer Herstellung sind bekannt [vgl. US 4 603 106, EP 260 610; EP 218 868 und Köhler und Milstein, Nature 256, 495-497 (1975); Current Topics in Microbiology and Immunology Vol. 81: Lymphocyte Hybridomas, Springer Verlag 1978; Monoclonal Antibodies, Kennet et al., Plenum Press 1980, S. 363-419].

Die pharmakologische Wirkung wurde am narkotisierten Schwein und Rhesusaffen gefunden, bei denen experimentell die Durchblutung reduziert wurde.

Zur vorliegenden Erfindung gehören pharmazeutische Zubereitungen, die neben nicht-toxischen, inerten pharmazeutisch geeigneten Trägerstoffen die erfindungsgemäßen Verbindungen enthalten, sowie Verfahren zur Herstellung dieser Zubereitungen.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

## Patentansprüche

1. Verwendung von anti-TNF-Antikörpern zur Herstellung von Arzneimitteln für die Behandlung von akutem Herzinfarkt.

## Claims

1. Use of anti-TNF antibodies for the preparation of medicaments for the treatment of acute cardiac infarct.

## Revendications

1. Utilisation d'anticorps anti-TNF pour la préparation de médicaments destinés au traitement de l'infarctus aigu du myocarde.
